# EUROPEAN PATENT APPLICATION

(11) **EP 3 047 870 A1**
(43) Date of publication of application: **27.07.2016**
(21) Application number: 16152116.6
(22) Date of filing: 20.01.2016
(51) Int. Cl.: A61M 25/02

(54) **DRESSING**

(30) Priority: 20.01.2015 GB 201500935
(71) Applicant: Greater Glasgow and Clyde NHS Board, Glasgow Strathclyde G12 0XH (GB)
(72) Inventor: MITCHELL, William, Glasgow, G11 7LJ (GB); LAWRENCE, Matthew, East Riding of Yorkshire, YO8 6EW (GB)
(74) Representative: HGF Limited

(57) **Abstract**

A dressing (1, 50) is presented comprising a base portion (2, "single layer") and at least one flap (12a, 12b, 54, 56) connected to the base portion; the base portion comprising an adhesive subject contacting surface (26, "adhesive patient contacting surface"); the at least one flap comprising an adhesive surface (20, "adhesive surface") and being adapted to be repositioned over a first edge (6, 68) of the dressing; wherein during use the base portion is fixed to the skin of a subject beneath a cannula inserted into the subject at an insertion point such that the first edge faces the insertion point, and the at least one flap is repositioned over the first edge and the inserted cannula and fixed to the skin of the subject to secure the cannula to the subject.

Methods of applying the dressing and kits comprising the dressing are also presented.

## Description

### Field of the Invention

The present invention relates to the field of dressings, more specifically to the field of dressings for securing a cannula to a patient.

### Background of the Invention

It is often necessary to administer fluids, blood products, or intravenous medications to patients, and this is typically carried out by inserting a cannula into a patient's vein (a peripheral cannula). It is common practice to withdraw blood samples from the cannula when it is first inserted. Arterial cannulas can also be inserted into an artery and these are often used in intensive care and high dependency units for invasive monitoring of blood pressure. Intravenous and arterial cannulas are therefore an important and necessary part of patient care.

Where cannulas are inserted into either a vein or an artery, a removable needle acts as a trocar allowing the skin and the wall of the vessel to be punctured, and guides the cannula into the correct position. The needle is withdrawn, removed and disposed of safely leaving the soft cannula tubing in place within the vessel. The cannula wings and access port remain outside on top of the skin.

Once inserted, the cannula must be secured to the patient to ensure that the cannula remains immobile and secure to prevent the cannula from being dislodged, and to minimise any pain and discomfort experienced by the patient.

A cannula is typically secured to the skin by an adhesive dressing that covers the cannula and fixes the cannula down. However, dressings used in the art can be difficult to apply, have a relatively large footprint (dressing surface area) and the application of the dressing to secure the cannula can cause discomfort. A common frustrating situation is the dressing becoming unstuck due to the dressing not covering the anchor point on the cannula, which results in the cannula becoming dislodge. Due to the surface area of the dressing being too big for the cannula, patient movement, washing, dressing etc can cause the dressing to become unsecure and unstuck. For example, the dressing may become tethered and crumpled, exposing the adhesive surface of the dressing, which may provide potential colony points for bacteria, thereby increasing the risks of infection. Sometimes the opposite may occur whereby the large adhesive area of the dressing can be difficult to remove. This can pose a serious problem, especially in those patients who have frail and paper-like thin skin, in particular the elderly and those who take certain medications, where the skin can be ripped or torn.

Therefore, there is a need for improved dressings that allow a cannula to be fixed to the body of a patient more securely, are easier to apply and remove, and to minimise any discomfort caused to the patient.

Accordingly, one object of the present invention is to provide an improved dressing for securing a cannula to a patient.

### Statements of the Invention

According to a first aspect of the invention there is presented a dressing comprising a base portion and at least one flap connected to the base portion; the base portion comprising an adhesive subject contacting surface; the at least one flap comprising an adhesive surface and being adapted to be repositioned over a first edge of the dressing; wherein during use the base portion is fixed to the skin of a subject beneath a cannula inserted into the subject at an insertion point such that the first edge is adjacent to the insertion point, and the at least one flap is repositioned over the first edge and the inserted cannula and fixed to the skin of the subject to secure the cannula to the subject.

Typically, a cannula is a tube that may be inserted into a subject to allow fluids to be delivered to the subject, or to allow fluids to be extracted from a subject. The cannula may be used to extract or feed liquids from/into a subject. For example, if blood samples are required to be extracted from a patient, a cannula may be inserted into the circulatory system of the patient via a vein or artery, and may be more convenient than repeated extraction of blood samples using syringes. When the cannula is to be inserted into a vein or artery, for example, the cannula may be attached to a needle (acting as a trocar) or similar, to facilitate insertion, and is secured in place with a dressing.

Dressings used in the art to secure cannulas to patients typically have a large footprint and therefore cover a relatively large surface area of the subject's skin, which can increase the risk of irritation and discomfort for the patient, and potentially make the dressing more difficult to remove. In addition, a cannula secured by dressings in the art are often not securely fastened to the patient and are too readily moved or completely removed by accident.

In addition, the inventors have surprisingly found that by encapsulating the cannula between the base portion and the at least one flap, the dressing of the present invention fastens the cannula more securely to the patient.

Furthermore, the provision of a dressing comprising at least one flap that is repositioned during use allows a cannula to be secured to a patient with a dressing having a significantly lower footprint than those known in the art.

Preferably, the at least one flap is adapted to be folded over the first edge of the dressing. Accordingly, repositioning of the at least one flap corresponds to the at least one flap being folded over the first edge of the dressing. Alternatively, the at least one flap may be adapted to be twisted or rotated over the first edge of the dressing, and repositioning of the at least one flap may correspond to twisting or rotating the at least one flap over the first edge of the dressing.

Preferably, the dressing comprises two flaps. The two flaps may be connected to the centre of the dressing and extend from the centre of the dressing in opposite directions.

Preferably, the two flaps are arranged on opposed sides of the base portion. For example, during use, the base portion may be in the middle of the dressing and adjacent the inserted cannula, and the two flaps may be arranged laterally of the base portion and of the inserted cannula.

Preferably, the two flaps at least partially overlap when repositioned over the first edge and the cannula, and secured to the skin of the subject.

The base portion may comprise at least one securing means configured to secure or retain a tube or cannula. For example, the at least one securing means may secure a tube such as a giving set extending from a feedbag to the cannula. The base portion may comprise at least two securing means.

The at least one securing means may be a loop of material that prevents a tube or similar that is threaded through the loop being pulled away from the patient, or from becoming unsecured from the patient. The at least one securing means may be a clip, a flap or similar. For example, the at least one securing means may be a flap of material that may be secured over a tube against the dressing. Typically, the tube may be a giving set tube that extends from the cannula to a feed bag. In embodiments where the base portion is a first layer of the dressing, two securing means may be arranged on either side of the dressing such that a tube, such as a feed tube, may be threaded through one of the two securing means whilst ensuring that the location of the tube is appropriate. For example, in embodiments where the dressing is applied to the right or left hand of a patient, the tube may be threaded through a securing means on the outside of the patient's hand, by using the appropriate securing means. Accordingly, the provision of two securing means provides extra flexibility for healthcare professionals.

In some embodiments, the dressing comprises a first layer comprising the base portion and the dressing comprises a second layer comprising the at least one flap. Preferably, the second layer comprises at least two flaps arranged either side of a cannula during use, and which at least partially overlap when repositioned over the cannula and secured to the skin of the subject. Typically, the first layer may be the bottom layer of the dressing during use, and therefore, the first layer may be a base layer. The second layer may be the top layer of the dressing once the dressing has been applied to a patient, and therefore, the second layer may be a top layer.

The base portion may comprise a removable cover that protects the adhesive subject contacting surface of the base portion and is removed before use. Typically, the removable cover may comprise a material that is readily removed from the adhesive subject contacting surface of the base portion without adversely affecting the adhesive properties of the adhesive subject contacting surface. The removable cover may comprise one or more tabs to allow the user to readily grip the removable cover to thereby remove the removable cover from the adhesive subject contacting surface of the base portion.

Typically, the base portion comprises a breathable material and suitable materials will be readily apparent to those skilled in the art. For example, in embodiments where the base portion is a first layer of the dressing, the first layer may comprise a breathable material.

The at least one flap may be transparent or semi-transparent. Therefore, the cannula and cannula insertion point where the cannula is anchored to the patient may be visible to a healthcare professional whilst the dressing is applied to a subject.

The adhesive surface of the at least one flap may be on the opposed side of the dressing to the adhesive subject contacting surface of the base portion. Accordingly, the at least one flap may be folded over the first edge of the dressing during use such that the adhesive surface is facing the skin of the subject.

Alternatively, the adhesive surface of the at least one flap may be on the same side of the dressing to the adhesive subject contacting surface of the base portion. Accordingly, the at least one flap may be twisted round over the first edge of the dressing during use such that the adhesive surface is facing the skin of the subject.

In embodiments where the at least one flap comprises an adhesive surface, the or each at least one flap may comprise a removable cover that protects the adhesive surface and is removed before use. Typically, the removable cover may comprise a material that is readily removed from the adhesive surface of the or each at least one flap without adversely affecting the adhesive properties of the adhesive surface. The removable cover may comprise one or more tabs to allow the user to readily grip the removable cover to thereby remove the removable cover from the adhesive surface of the or each at least one flap.

Accordingly, the dressing may be easier to apply to a subject and the dressing may be applied more quickly than those known in the art.

Preferably, the first edge of the dressing comprises a cannula receiving element adapted to receive a cannula during use. The cannula receiving element may be a recess that is configured to receive the cannula and to allow the dressing to extend at least partially around an inserted cannula at or adjacent to the point of insertion of the cannula. Alternatively, the cannula receiving element may be a notch within the dressing, or a slot within the dressing. Typically, in embodiments where the dressing comprises a first layer and a second layer, the cannula receiving element may extend across both the first and second layers.

The cannula receiving element may be tapered. The cannula receiving element may be uniform. For example, the width of the cannula receiving element may be tapered, or the width of the cannula receiving element may be uniform.

Typically, the cannula receiving element allows the dressing to extend around the cannula and thereby allowing the dressing to secure the cannula to the skin of the patient close to the insertion point where the cannula enters the patient, without requiring the cannula to be lifted up to allow the dressing to be inserted underneath the cannula. Therefore, the dressing of the present invention may allow the cannula to be more securely, or at least as securely, fixed to the patient without requiring substantial lifting of the cannula, which may result in discomfort or pain for the patient, or in the cannula breaking.

Preferably, during use, the at least one flap is fixed to the subject such that the at least one flap covers the point at which the cannula is anchored to the patient. Typically, the point at which the cannula is anchored to the patient corresponds to the point at which the cannula has been inserted into the cannulated subject. Accordingly, the at least one flap provides support to the anchor point of the cannula to thereby more securely retain the cannula within the cannulated subject.

In some embodiments of the invention, the dressing may comprise a label. In embodiments where the dressing comprises a first layer and a second layer, the first layer may comprise a label, and/or the second layer may comprise a label. The label may be an area on the top surface of the dressing that is exposed once the dressing has been applied to secure a cannula to a patient. The area may comprise a material adapted to allow the healthcare professional applying the dressing to write on the dressing information relevant to the patient and the procedure they have or may be about to undergo. For example, the healthcare professional may write the date the cannula was inserted, the type of cannula inserted, or the fluid being administered to or extracted from the patient.

The at least one flap may comprise a recess, and the recess of the at least one flap and the cannula receiving element may be configured to define an aperture when the at least one flap is folded over the cannula, and through which the cannula may extend once the dressing has been applied.

The recess of the at least one flap may be shaped to comprise a curve or an angular element. For example, in embodiments where the cannula has a circular cross-section, the recess of the at least one foldable element may be curved to accommodate the cannula.

The provision of a recess in the at least one flap ensures that the at least one flap may be repositioned over the cannula with the recess abutting the cannula, for example, without any adhesive of the adhesive surface being exposed, or minimizing the amount of adhesive of the adhesive surface being exposed, and allows the cannula to be more secure, and minimizes the number of potential bacterial colony sites.

Preferably, the at least one flap is connected to the base portion by a flexible portion associated with the or each at least one flap, such that the or each at least one flap is repositioned with a change in configuration of the flexible portion during use.

The flexible portion may be a living hinge where the material of the dressing is thinner or otherwise more flexible in the flexible portion than in either the base portion or the at least one flap. The flexible portion may be a crease or perforation of the material of the dressing, and is therefore more flexible than the material of the base portion and the flap.

Preferably, in embodiments where the at least one flap is folded over the first edge, the flexible portion determines the angle at which the at least one flap is folded during use. Accordingly, the angle of folding is consistent between dressings and is not solely determined by the user.

In a preferred embodiment, the dressing of the invention is oblong having a major dimension and a minor dimension, and when used to secure a cannula, the major dimension of the dressing is arranged substantially perpendicular to the length of the cannula. The base portion may correspond to a central portion with a first flap and a second flap arranged either side of the central portion. Typically, the flaps extend from the central portion along the major dimension of the dressing towards opposed ends of the dressing. During use the at least one flap is folded at an angle to the major dimension of the dressing. The angle to the major dimension of the dressing may be greater than about 45°, 50°, 60° or 70°. The angle to the major dimension of the dressing may be between about 45°-80°. In embodiments comprising a flexible portion associated with the or each flap, the angle at which the at least one flap is folded is determined by the or each flexible portion.

In embodiments where the dressing comprises two flaps, a first flexible portion is associated with the first flap, and a second flexible portion is associated with the second flap, the first flap may extend from first flexible portion towards a first end of the dressing, and the second flap may extend from the second flexible portion towards a second end of the dressing. The first and second flexible portions may be arranged in the central portion of the dressing and the first and second flaps may extend away from the central portion towards or to the first and second ends of the dressing. In embodiments where the dressing comprises a first layer, the first and second flaps may extend beyond the first layer.

The invention extends in a second aspect to a method of securing a cannula of a cannulated subject, the method comprising the steps of:
i) providing a dressing according to the first aspect of the invention;
ii) inserting the dressing between the cannula and the skin of the subject;
iii) fixing the base portion of the dressing to the patient; and
iv) repositioning the at least one flap over the cannula and fixing the at least one flap to the subject;
wherein the cannula is thereby secured to the subject.

Preferably, step iv) comprises folding the at least one flap over the cannula.

Typically, the dressing is fixed to the skin of the subject. Typically, the at least one flap is fixed to the skin of the subject.

Preferably, the at least one flap is fixed to the subject such that the at least one flap covers the point at which the cannula is anchored to the patient. Typically, the point at which the cannula is anchored to the patient corresponds to the point at which the cannula has been inserted into the cannulated subject. Accordingly, the at least one flap provides support to the anchor point of the cannula to thereby more securely retain the cannula within the cannulated subject.

The base portion may comprise a removable cover that protects the adhesive subject contacting surface of the base portion and the method may comprise the step of removing the removable cover to expose the adhesive subject contacting surface prior to step iii).

Preferably, the or each at least one flap comprises an adhesive surface. Preferably, the or each at least one flap comprises a removable cover that protects the adhesive surface, and the method comprises the step of removing the removable cover to expose the adhesive surface prior to step iv).

Preferably, the dressing comprises two flaps corresponding to a first foldable element, and a second foldable element, wherein, the first and second foldable elements at least partially overlap when they are folded over the first edge and the cannula.

Preferably, the first edge of the dressing comprises a cannula receiving element, and step iii) further comprises inserting the cannula into the cannula receiving element.

According to a third aspect of the invention there is presented a kit of parts comprising a plurality of dressings according to the first aspect of the invention.

The kit may comprise instructions directing a user to apply a dressing from the plurality of dressings according to the method of the second aspect. The instructions may be written on an information sheet or similar, for example.

The kit may comprise one or more cannulas and the plurality of dressings may be suitably dimensioned to secure the or each cannula to a subject. Preferably, each dressing within the plurality of dressings comprises a cannula receiving element adapted to receive the cannula of the kit. For example, the kit may comprise one or more cannulas of an appropriate size for use with adult subjects and the plurality of dressings may be suitably dimensioned to secure the or each cannula to an adult subject. The kit may comprise one or more cannulas of an appropriate size for use with infant subjects and the plurality of dressings may be suitably dimensioned to secure the or each cannula to an infant subject.

Optional and preferred features of the first aspect are optional and preferred features of the second and third aspects.

The invention extends in a fourth aspect to a dressing for securing a cannula to a subject as depicted in the Figures.

Embodiments of the present invention will now be described, by way of non-limiting example, with reference to the accompanying drawings.

### Brief Description of the Figures

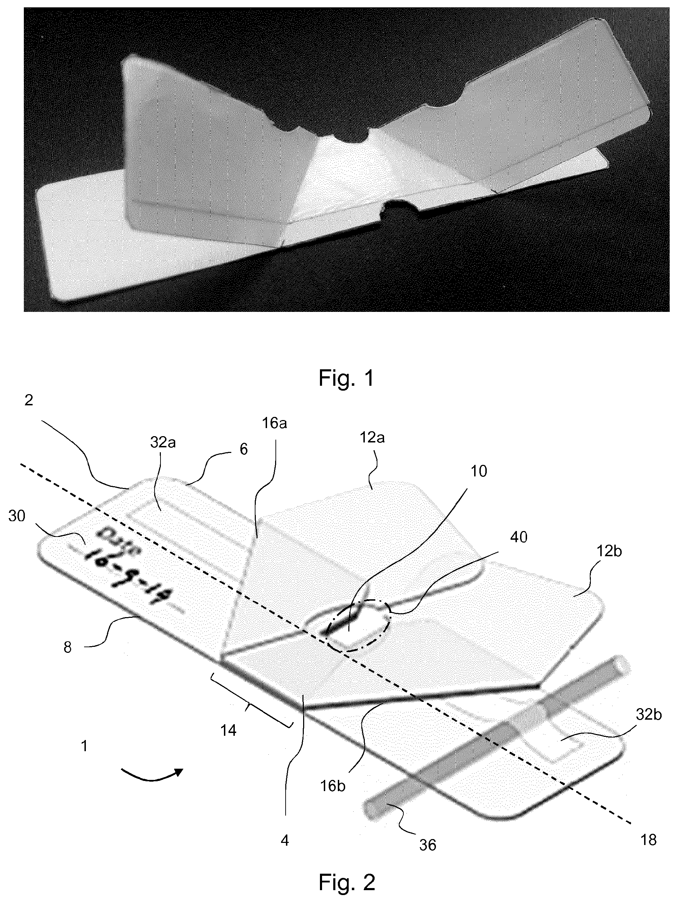

Figure 1 shows a perspective view of an embodiment of the invention;
Figure 2 shows a schematic perspective view of an embodiment of the invention;

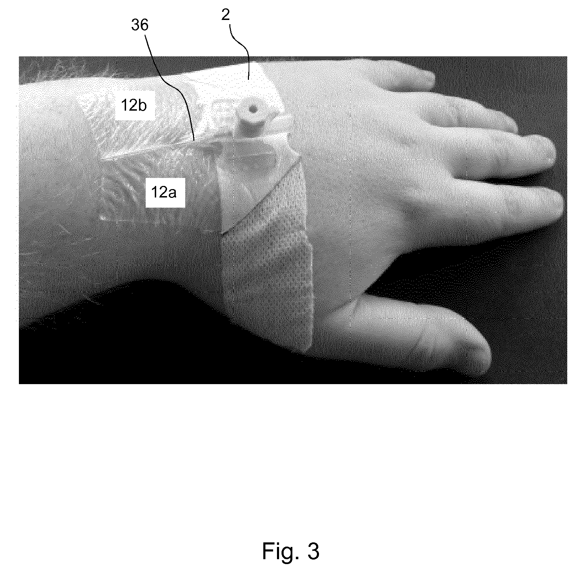

Figure 3 shows a photograph of an embodiment of the invention securing a cannula to the back of the hand of a patient;
Figure 4 shows a series of images of an embodiment of the invention illustrating how that embodiment may be used to secure a cannula to a patient;

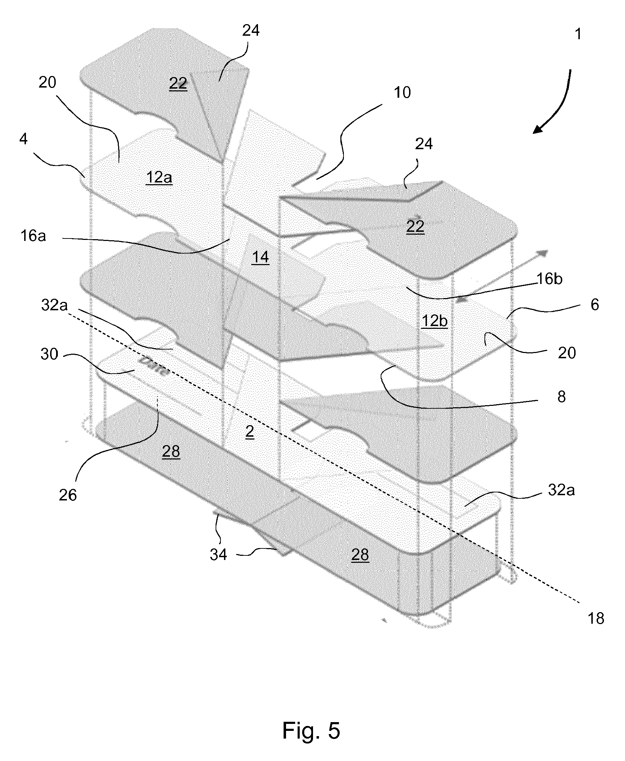

Figure 5 shows an exploded perspective view of an embodiment of the invention; and

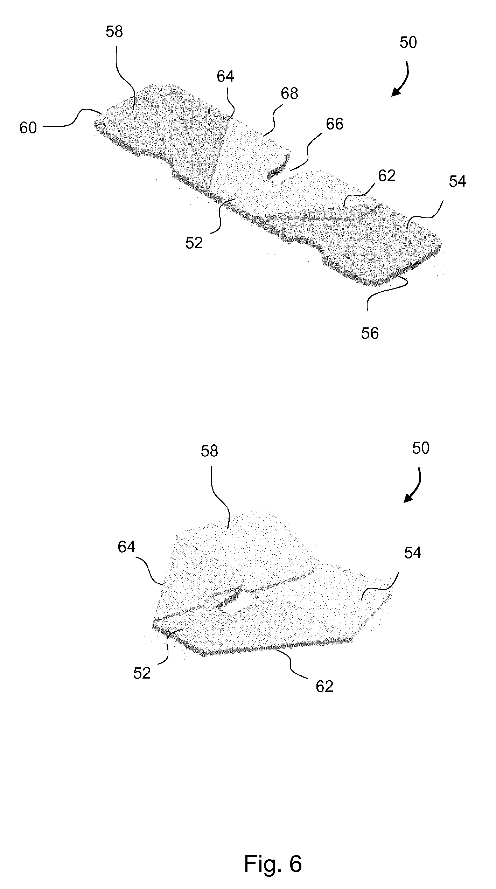

Figure 6 shows a perspective view of an embodiment of the invention.

### Specific Description of Embodiments of the Invention

While the making and using of various embodiments of the present invention are discussed in detail below, it should be appreciated that the present invention provides many applicable inventive concepts that can be embodied in a wide variety of specific contexts. The specific embodiments discussed herein are merely illustrative of specific ways to make and use the invention and do not delimit the scope of the invention.

To facilitate the understanding of this invention, a number of terms are defined below. Terms defined herein have meanings as commonly understood by a person of ordinary skill in the areas relevant to the present invention. Terms such as "a", "an" and "the" are not intended to refer to only a singular entity, but include the general class of which a specific example may be used for illustration. The terminology herein is used to describe specific embodiments of the invention, but their usage does not delimit the invention, except as outlined in the claims.

### First Example Embodiment

In an example embodiment of the invention, with reference to the Figures, an oblong dressing 1 comprises a base dressing 2 of breathable fabric (acting as a base portion), a top layer 4, a first edge 6, an opposed second edge 8, and a notch 10 in the centre of the first edge (acting as a cannula receiving element).

The top layer comprises a first wing 12a and a second wing 12b (both acting as flaps), and a central connecting portion 14. The first wing and the second wing are connected to the central connecting portion on opposing sides by a first and second living hinge 16a, 16b (acting as flexible portions), and the first wing and second wing extend away from the central connecting portion along the major dimension of the dressing 18. The first and second wings comprise an adhesive top surface 20 and the adhesive top surface is covered by non-stick paper 22, and the opposed bottom surface of the first and second wings is covered by protective paper 23. The non-stick paper comprises a tab 24 to allow the user to more readily grip and then remove the non-stick paper during use. The first living hinge determines the angle to the major dimension of the dressing at which the first wing is folded during use, and the second living hinge determines the angle to the major dimension of the dressing at which the second wing is folded during use. The first and second wings each further comprise a recess 25a, 25b on the second edge of the dressing before use. That is, the recess is on the second edge of the dressing before the first and second wings are folded to cover the cannula.

The base dressing comprises an adhesive bottom surface 26 (acting as an adhesive surface) covered by non-stick paper 28, a label 30 and a first and a second holder (acting as securing means) 32a, 32b on the top surface, one holder either side of the notch in the first edge. The non-stick paper comprises a tab 34 to allow the user to more readily grip and remove the non-stick paper.

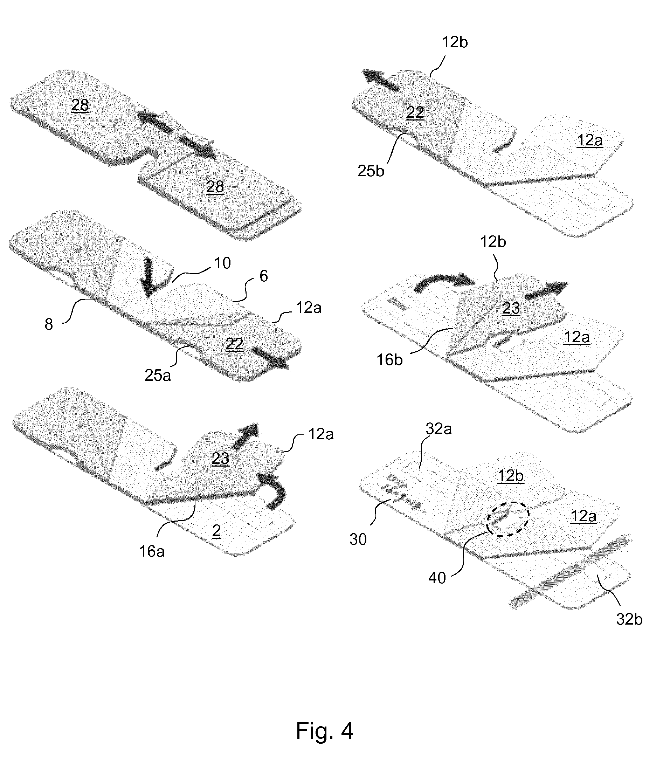

During use, a cannula 36 is inserted into a patient. With reference to Figure 4, the non-stick paper on the adhesive bottom surface of the base dressing is removed and the dressing fixed to the skin of the patient beneath the portion of the cannula extending from the skin of the patient. The first edge of the dressing is proximal to the insertion point 38, and the second edge of the dressing is distal to the insertion point, and the cannula is inserted into the notch in the first edge such that the dressing extends around the cannula. Therefore, the cannula rests on the dressing but is not as yet secured to the dressing.

The non-stick paper on the first wing is then removed and the first wing is folded along the first living hinge such that the first side wing extends towards the insertion point of the cannula, over the first edge of the dressing, and over the cannula. The first wing is then secured to the skin of the patient, and in the process, is secured to the cannula, thereby securing the cannula to the patient. The protective paper is then removed from the first wing.

In a similar way, the non-stick paper on the second wing is then removed and the second wing is folded along the second living hinge such that the second wing extends towards the insertion point of the cannula, over the first edge of the dressing, and over the cannula. The second wing is then secured to the skin of the patient, and in the process, is secured to the cannula, thereby securing the cannula to the patient. The protective paper is then removed from the second wing.

Once secured, the cannula extends through an aperture 40 defined by the notch in the first edge of the dressing and the adjacent edges of the first wing and the second wing.

A tube such as a giving set extending from the cannula to a feed bag may then be retained in the first or second holder to prevent the tube (and thereby the cannula) being accidentally snagged by the patient, for example, and dislodging the cannula.

The healthcare professional applying the dressing to the patient may then use the label on the base dressing to record the time and/or date that the dressing was applied to ensure that it is readily apparent the length of time that the dressing has been in place.

### Second Example Embodiment

A dressing 50 comprises a single layer, the single layer comprising a patient contacting surface in a central portion 52 (acting as a base portion), and a first foldable portion 54 (acting as a first flap) arranged on a first side 56 of the central portion and a second foldable portion 58 (acting as a second flap) arranged on the opposed second side 60 of the central portion. The patient contacting surface comprises adhesive to thereby fix the dressing to the skin of a patient during use (thereby acting as an adhesive subject contacting surface). The first foldable portion is connected to the central portion by a first flexible portion 62 and the second foldable portion is connected to the central portion by a second flexible portion 64. The first foldable portion and the second foldable portion comprise an adhesive surface, and the adhesive surface is opposed to the patient contacting surface. The central portion comprises a label (not shown) and a slot 66 on a first edge of the dressing 68.

During use, the dressing is placed between an inserted cannula and the skin of a subject such that the slot on the first edge is around the cannula, and fixed in place by applying pressure to the central portion of the dressing. The first foldable portion is then folded at the first flexible portion over the first edge and the cannula, such that the adhesive surface of the first foldable portion is facing the skin of the subject, and pressure is applied to the first foldable portion to fix the first foldable portion to the skin of the subject. The second foldable portion is then folded at the second flexible portion over the first edge and the cannula, such that the adhesive surface of the second foldable portion is facing the skin of the subject, and pressure is applied to the second foldable portion to fix the second foldable portion to the skin of the subject.

Accordingly, the first foldable portion and the second foldable portion overlap over the cannula, thereby securing the cannula to the subject.

In an alternative embodiment, the adhesive patient contacting surface is protected with a cover of "non-stick" paper and the cover has a tab. During use, the user grips the tab and removes the cover before placing and fixing the dressing between the cannula and the skin of the subject. The adhesive surface of the first foldable portion and the second foldable portion are each protected by a cover of non-stick paper, each of which has a tab. During use, for each foldable portion the user grips the tab and removes the cover before that foldable portion is folded over the first edge and the cannula fixed to the skin of the subject.

The invention encompasses any and all possible combinations of some or all of the various embodiments described herein. It should also be understood that various changes and modifications to the presently preferred embodiments described herein will be apparent to those skilled in the art. Such changes and modifications can be made without departing from the spirit and scope of the invention and without diminishing its intended advantages. It is therefore intended that such changes and modifications be covered by the appended claims.

## Claims

1. A dressing comprising a base portion and at least one flap connected to the base portion; the base portion comprising an adhesive subject contacting surface; the at least one flap comprising an adhesive surface and being adapted to be repositioned over a first edge of the dressing; wherein during use the base portion is fixed to the skin of a subject beneath a cannula inserted into the subject at an insertion point such that the first edge is adjacent to the insertion point, and the at least one flap is repositioned over the first edge and the inserted cannula, and fixed to the skin of the subject to secure the cannula to the subject.

2. The dressing according to claim 1, wherein the at least one flap is adapted to be folded over the first edge of the dressing, and wherein the dressing comprises a first layer comprising the base portion and the dressing comprises a second layer comprising the at least one flap.

3. The dressing according to any preceding claim, wherein the adhesive surface of the at least one flap is on the opposite side of the dressing to the adhesive subject contacting surface of the base portion before the at least one flap is repositioned and the adhesive surface of the at least one flap is on substantially the same side of the dressing as the adhesive subject contacting surface of the base portion after the at least one flap is repositioned.

4. The dressing according to any preceding claim, wherein the dressing comprises two flaps, wherein the two flaps are connected to the centre of the dressing and extend from the centre of the dressing in opposite directions, and wherein the two flaps at least partially overlap when repositioned over the first edge and the cannula, and secured to the skin of the subject.

5. The dressing according to any preceding claim, wherein the base portion comprises at least one securing means configured to secure or retain a tube.

6. The dressing according to any preceding claim, wherein the first edge of the dressing comprises a cannula receiving element adapted to receive a cannula during use.

7. The dressing according to claim 6, wherein the cannula receiving element is a recess that is configured to receive the cannula and to allow the dressing to extend at least partially around an inserted cannula at or adjacent to the point of insertion of the cannula.

8. The dressing according to any preceding claim, wherein the at least one flap comprises a recess, and the recess of the at least one flap and the cannula receiving element are configured to define an aperture when the at least one flap is folded over the cannula, and through which the cannula extends once the dressing has been applied.

9. The dressing according to any preceding claim, wherein the at least one flap is connected to the base portion by a flexible portion associated with the or each at least one flap, such that the or each at least one flap is repositioned with a change in configuration of the flexible portion during use.

10. A method of securing a cannula of a cannulated subject, the method comprising the steps of:
i) providing a dressing according to any of claims 1 to 9;
ii) inserting the dressing between the cannula and the skin of the subject;
iii) fixing the base portion of the dressing to the patient; and
iv) repositioning the at least one flap over the cannula and fixing the at least one flap to the subject;
wherein the cannula is thereby secured to the subject.

11. The method according to claim 10, wherein step iv) comprises folding the at least one flap over the cannula.

12. The method according to either of claims 10 or 11, wherein the base portion comprises a removable cover that protects the adhesive surface of the base portion and the method comprises the step of removing the removable cover to expose the adhesive surface prior to step iii).

13. The method according to any of claims 10 to 12, wherein the first edge of the dressing comprises a cannula receiving element, and step iii) further comprises inserting the cannula into the cannula receiving element.

14. A kit of parts comprising a plurality of dressings according to any of claims 1 to 9 and instructions directing a user to apply a dressing from the plurality of dressings according to the method of any one of claims 10 to 13.

15. A kit according to claim 14, wherein the kit comprises one or more cannulas and the plurality of dressings are suitably dimensioned to secure the or each cannula to a subject, wherein each dressing within the plurality of dressings comprises a cannula receiving element dimensioned to receive the cannula of the kit.
